# EUROPÄISCHE PATENTSCHRIFT

(11) **EP 1 185 253 B1**
(45) Veröffentlichungstag und Bekanntmachung des Hinweises auf die Patenterteilung: **03.03.2004**
(21) Anmeldenummer: 00942052.2
(22) Anmeldetag: 13.06.2000
(51) Int. Cl.: A61K 9/48, A61K 9/16, A61K 31/135, A61K 31/195, A61K 9/20, A61K 9/50

(54) **ORALE DARREICHUNGSFORMEN ZUR VERABREICHUNG EINER FIXEN KOMBINATION VON TRAMADOL UND DICLOFENAC**
ORAL ADMINISTRATION FORM FOR ADMINISTERING A FIXED TRAMADOL AND DICLOFENAC COMBINATION
FORME GALENIQUE ORALE SERVANT A ADMINISTRER UNE COMBINAISON FIXE DE TRAMADOL ET DE DICLOFENAC

(30) Priorität: 17.06.1999 DE 19927689
(43) Veröffentlichungstag der Anmeldung: 13.03.2002
(73) Patentinhaber: Grünenthal GmbH, 52078 Aachen (DE)
(72) Erfinder: BARTHOLOMÄUS, Johannes, D-52080 Aachen (DE); ZIEGLER, Iris, D-52159 Rott-Roetgen (DE)
(86) Internationale Anmeldenummer: PCT/EP2000/005386
(87) Internationale Veröffentlichungsnummer: WO 2000/078294

(56) Entgegenhaltungen:
- EP-A- 0 546 676
- WO-A-98/17268
- DATABASE CHEMABS [Online] CHEMICAL ABSTRACTS SERVICE, COLUMBUS, OHIO, US; STANISLAVCHUK, N. A. ET AL: "Antinociceptive effect of tramal in combination with nonsteroidal antiinflammatory compounds" retrieved from STN Database accession no. 127:215039 HCA XP002156648 & EKSP. KLIN. FARMAKOL. (1997), 60(1), 22-24 ,

## Beschreibung

Die vorliegende Erfindung betrifft eine orale Applikationseinheit, enthaltend die Wirkstoffe Tramadol und Diclofenac und/oder deren jeweilige physiologisch verträgliche Salze, wobei die beiden Wirkstoffe in jeweils separat formulierten Untereinheiten in derselben Applikationseinheit vorliegen.

Tramadol stellt ein Analgetikum zur Behandlung starker und mittelstarker Schmerzen dar, dessen Wirkprinzip nicht auf einem reinen Opioid-Mechanismus beruht. Tramadol zeigt auch nicht die für ein Opioid charakteristichen Nebenwirkungen. In einigen Fällen wird als unerwünschte Begleiterscheinung Übelkeit beobachtet.

Ebenfalls bekannte, nicht opioide, zur Bekämpfung weniger intensiver Schmerzen geeignete Analgetika sind steroidfreie Schmerzmittel wie Diclofenac-Na, Acetylsalicylsäure oder Ibuprofen.

Desweiteren wird von der WHO für die Behandlung von mäßig bis starken Schmerzen empfohlen, opioide Schmerzmittel mit nicht sterioiden Schmerzmitteln zu kombinieren, um eine effektivere Schmerzlinderung herbeizuführen und gegebenfalls die benötigten Mengen zu verringern.

Das europäische Patent EP -B- 0 546 676 lehrt zum Beispiel, daß die Kombination von Tramadol-HCl mit steroidfreien entzündungshemmenden Mitteln wie zum Beispiel Ibuprofen in einer Zusammensetzung von 1:1 bis 1:200 zu einer synergistisch verstärkten analgetischen Wirkung führt. Tramadol-HCl und Diclofenac-Na bilden jedoch eine schwerlösliche Verbindung. Damit ist zu erwarten, daß die Bioverfügbarkeit der beiden Wirkstoffe reduziert wird und zum Ausgleich höhere Dosierungen notwendig werden.

Aufgabe der vorliegenden Erfindung war es daher, die beiden Wirkstoffe Tramadol und Diclofenac bzw. deren jeweils physiologisch verträgliche Salze in einer gemeinsamen Applikationseinheit zu kombinieren, ohne daß
es zu einer Beeinträchtigung der Freisetzungsprofile beider Wirkstoffe und zu einer Verringerung der Bioverfügbarkeit kommen kann.

Erfindungsgemäß wird diese Aufgabe durch die Bereitstellung einer oralen Applikationseinheit gelöst, die die beiden Wirkstoffe Tramadol und Diclofenac und/oder deren jeweilige physiologisch verträgliche Salze enthält, wobei aber die beiden Wirkstoffe jeweils in separat formulierten Untereinheiten in derselben Applikationseinheit enthalten sind.

Vorzugsweise enthalten die Untereinheiten als physiologisch verträgliche Salze des Tramadols Tramadolhydrochlorid, Tramadolhydrobromid, Tramadolsulfat, Tramadolphosphat, Tramadolfumarat, Tramadolsuccinat, Tramadolmaleat, Tramadolnitrat, Tramadolacetat, Tramadolpropionat, Tramadolmalonat, Tramadolcitrat, Tramadoltartrat, Tramadolbenzoat, Tramadolsalicylat, Tramadolphthalat und/oder Tramadolnicotinat. Besonders bevorzugt enthalten die Untereinheiten Tramadolhydrochlorid.
Vorzugsweise enthalten die Untereinheiten als physiologisch verträgliche Salze des Diclofenacs Diclofenac-Natrium, Diclofenac-Kalium, Diclofenac-Kalzium, Diclofenac-Magnesium und/oder Diclofenac-Colestyramin. Besonders bevorzugt enthalten die Untereinheiten Diclofenac-Natrium.

Vorzugsweise enthält die orale Applikationseinheit die Wirkstoffe Tramadol und Diclofenac in einem Mengenverhältnis von ≤1:4 bis 4:≤1, besonders bevorzugt im Mengenverhältnis von 0,5:1 bis 3:1 und ganz besonders bevorzugt im Mengenverhältnis von 1:1 bis 2,5:1.

Bei den Untereinheiten im Sinne der Erfindung handelt es sich um feste Arzneimittelformulierungen, die neben dem jeweiligen Wirkstoff und/oder seinen jeweiligen physiologisch verträglichen Salzen die üblichen Hilfs- und Zusatzstoffe enthalten.

Vorzugsweise liegen die Untereinheiten in multipartikulärer Form vor, wie z.B. als Mikrotabletten, Mikrokapseln, lonenaustauscherresinate, Granulate, Wirkstoffkristalle oder Pellets. Besonders bevorzugt liegen die Untereinheiten in Form von Granulaten, Wirkstoffkristallen oder Pellets vor. Ganz besonders bevorzugt umfaßt die Form der Untereinheiten durch Extrusion und/oder Spheronisation hergestellte Pellets oder Aufbaupellets.

Die orale Applikationseinheit kann auch mindestens einen der beiden Wirkstoffe in einer retardierten, gegebenenfalls multipartikulären Form, bevorzugt beide Wirkstoffe in einer retardierten, gegebenenfalls multipartikulären Form enthalten.

Die orale Applikationseinheit kann auch mindestens einen der Wirkstoffe zusätzlich zu seiner retardierten Form auch in der nicht retardierten Form enthalten. Durch Kombination mit dem sofort freigesetzten Wirkstoff läßt sich eine schnelle Schmerzlinderung erzielen und die langsame Freisetzung aus der retardierten Form ermöglicht die Aufrechterhaltung therapeutischer Blutspiegel über längere Zeit. Besonders bevorzugt wird die Freisetzung der Wirkstoffe so einzustellen sein, daß die orale Applikationseinheit höchstens zweimal, vorzugsweise nur einmal täglich verabreicht werden muß. Dem Fachmann ist aufgrund der Wirkung der Analgetika bekannt, in welchen Mischungsverhältnissen diese einzusetzen sind, damit die gewünschte Wirkung erreicht wird.

Das Freisetzungsprofil der oralen Applikationseinheiten ist vorzugsweise so zu steuern, daß das Tramadol bzw. das Diclofenac bei zweimaliger Applikation pro Tag innerhalb von 8 Stunden ≥ 70 Gew.% bzw. ≥ 60 Gew.% freigesetzt wird. Gegenstand der Erfindung sind daher auch orale Applikationseinheiten für eine zweimalige Applikation pro Tag, die dadurch gekennzeichnet sind, daß das Tramadol bzw. das Diclofenac innerhalb von 8 Stunden ≥ 70 Gew.% bzw. ≥ 60 Gew. % freigesetzt wird.

Bei einmaliger Applikation pro Tag ist das Freisetzungsprofil vorzugsweise so zu steuern, daß innerhalb von 16 Stunden das Tramadol bzw. das Diclofenac ≥ 70 Gew.% bzw. ≥ 60 Gew.% freigesetzt wird.
Ein weiterer Gegenstand der Erfindung sind daher auch orale Applikationseinheiten für eine einmalige Applikation pro Tag, die dadurch gekennzeichnet sind, daß das Tramadol bzw. das Diclofenac innerhalb von 16 Stunden ≥ 70 Gew. % bzw. ≥ 60 Gew.% freigesetzt wird.
Bei oralen Applikationseinheiten, die multipartikuläre Untereinheiten mit magensaftresistenten Überzügen enthalten oder die selbst magensaftresistente Überzüge aufweisen, ist den genannten Freisetzungsprofilen bezüglich des Tramadols außerdem die Verweilzeit im Magen hinzuzurechnen.

Die Retardierung der jeweiligen Wirkstoffe in den jeweiligen Untereinheiten kann vorzugsweise durch einen retardierenden Überzug, Fixierung an einem Ionenaustauscherharz, Einbettung in eine retardierende Matrix oder einer Kombination daraus erfolgen.

Vorzugsweise wird die Retardierung mit Hilfe von retardierenden Überzügen erreicht. Geeignete, retardierende Überzüge umfassen wasserunlösliche Wachse oder Polymere, wie z.B. Acrylharze, vorzugsweise Poly(meth)acrylate, oder wasserunlösliche Cellulosen, vorzugsweise Ethylcellulose. Diese Materialien sind aus dem Stande der Technik, z.B. Bauer, Lehmann, Osterwald, Rothgang "Überzogene Arzneiformen", Wissenschaftliche Verlagsgesellschaft mbH Stuttgart, 1988, Seite 69 ff., bekannt. Sie werden hiermit als Referenz eingeführt

Neben den wasserunlöslichen Polymeren können gegebenenfalls zur Einstellung der Freisetzungsgeschwindigkeit des Wirkstoffes die Retardüberzüge auch nicht retardierende, vorzugsweise wasserlösliche Polymere vorzugsweise in Mengen bis 30 Gew.%, wie Polyvinylpyrrolidon oder wasserlösliche Cellulosen, vorzugsweise Hydroxypropylmethylcellulose oder Hydroxypropylcellulose, und/oder hydrophile Porenbildner, wie Saccharose, Natriumchlorid oder Mannitol und/oder die bekannten Weichmacher enthalten.
Außerdem können die multipartikulären Untereinheiten noch weitere Überzüge aufweisen. Als Überzüge können auch solche vorhanden sein, die sich pH-abhängig auflösen. So kann erreicht werden, daß die Untereinheiten den Magentrakt unaufgelöst passieren und erst im Darmtrakt zur Freisetzung gelangen. Es können auch Überzüge verwendet werden, die der Verbesserung des Geschmackes dienen.

Eine weitere übliche Verfahrensweise der Retardierung ist die Fixierung der Wirkstoffe an Ionenaustauscherharzen. Zur Retardierung des Wirkstoffes Diclofenac wird als anionisches Ionenaustauscherharz vorzugsweise Colestyramin eingesetzt. Zur Retardierung des Wirkstoffes Tramadol werden als kationisches Ionenaustauscherharz vorzugsweise Polystyrolsulfonate eingesetzt.

Zur Retardierung können die Untereinheiten die Wirkstoffe auch in einer retardierenden Matrix, vorzugsweise gleichmäßig verteilt, vorliegen.
Als Matrixmaterialien können physiologisch verträgliche, hydrophile Materialien verwendet werden, welche dem Fachmann bekannt sind. Vorzugsweise werden als hydrophile Matrixmaterialien Polymere, besonders bevorzugt Celluloseether, Celluloseester und/oder Acrylharze verwendet. Ganz besonders bevorzugt werden als Matrixmaterialien Ethylcellulose, Hydroxypropylmethylcellulose, Hydroxypropylcellulose, Hydroxymethylcellulose, Poly(meth)acrylsäure und/oder deren Derivate, wie deren Salze, Amide oder Ester eingesetzt.

Ebenfalls bevorzugt sind Matrixmaterialien aus hydrophoben Materialien, wie hydrophobe Polymere, Wachse, Fette, langkettigen Fettsäuren, Fettalkohole oder entsprechenden Ester oder Ether oder deren Gemische. Besonders bevorzugt werden als hydrophobe Materialien Mono- oder Diglyceride von C₁₂-C₃₀-Fettsäuren und/oder C₁₂-C₃₀-Fettalkohole und/oder Wachse oder deren Gemsiche eingesetzt.

Es ist auch möglich, Mischungen der genannten hydrophilen und hydrophoben Materialien als retardierendes Matrixmaterial einzusetzen.

Vorzugsweise ist die Darreichungsform der erfindungsgemäßen oralen Applikationseinheit ein Sachet, eine Kapsel oder eine Tablette, besonders bevorzugt eine Kapsel oder eine Tablette. Vorzugsweise ist die Tablette eine Pellettablette, die besonders bevorzugt schnell zerfällt.

Dazu kann die Tablette beim Kontakt mit wässrigen Medien wieder in die Untereinheiten zerfallen und die Wirkstoffe räumlich getrennt voneinander freisetzen. Als Sprengmittel, die die Untereinheiten bei Kontakt mit wäßrigen Medien voneinander trennen, können Crospovidone, Croscarmelose, Stärke und/oder niedrig substituierte Hydroxypropylcellulose verwendet werden.

Vorzugsweise weist die erfindungsgemäße Applikationseinheit als Tablette mindestens eine Bruchkerbe auf, die eine Teilung der Dosis, bevorzugt deren Halbierung ermöglicht. Dies ermöglicht ein Anpassung an die individuellen Bedürfnisse des Patienten, entsprechend der individuell zu verabreichenden Menge der Analgetika.

Die Herstellung der multipartikulären Untereinheiten, sowie der erfindungsgemäßen oralen Applikationseinheit kann nach den verschiedenen dem Fachmann bekannten Methoden erfolgen. Diese Methoden sind aus dem Stande der Technik, z.B. "Pharmaceutical Pelletization Technology", Drugs and the Pharmaceutical Sciences Vol 37, Verlag Marcel Dekker, bekannt. Sie werden hiermit als Referenz eingeführt. Sofern die erfindungsgemäße orale Applikationseinheit, wie z.B. die Tablette Überzüge aufweist, können diese nach üblichen Verfahren, wie z.B. Dragieren, Aufsprühen von Lösungen, Schmelzen, Dispersionen oder Suspensionen, durch Schmelzverfahren oder durch Pulverauftragsverfahren aufgebracht werden.

Diese Überzüge können retardierend oder nicht retardierend sein. Retardierende Überzüge bestehen aus den obengenannten Materialien. Neben dem retardierenden Überzug kann die erfindungsgemäße orale Applikationseinheit mindestens einen weiteren Überzug aufweisen. Ein solcher Überzug kann sich zum Beispiel pH-abhängig auflösen. So kann erreicht werden, daß die orale Applikationseinheit den Magentrakt unaufgelöst passiert und erst im Darmtrakt zur Freisetzung gelangt. Ein weiterer Überzug kann auch der Verbesserung des Geschmackes dienen.

Die Freisetzungsprofile der gemäß den Beispielen hergestellten erfindungsgemäßen Präparaten wurde wie folgt bestimmt:

Die Zubereitungen wurden entweder in einem Drehkörbchenapparat (Beispiele 1 und 3) oder in einer Blattrührapparatur (Beispiele 2 und 4) gemäß Europäischem Arzneibuch bei einer Temperatur von 37°C und einer Umdrehungsgeschwindigkeit von 100 min⁻¹ (Beispiele 1 und 3) bzw. 50 min⁻¹ (Beispiele 2 und 4) 2 Stunden lang in 600 ml künstlichem Magensaft ohne Enzyme (pH 1,2) gegeben. Anschließend wurden die Zubereitungen weitere 8 (Beispiel 3: weitere 6) Stunden lang in 900 ml künstlichem Darmsaft ohne Enzyme (pH 7,2) gegeben. Dieser pH-Wert wurde bis zu der Untersuchung beibehalten. Die jeweils zu einem Zeitpunkt freigesetzte Menge des jeweiligen Wirkstoffes Tramadol oder Diclofenac wurde durch HPLC bestimmt. Die dargestellten Werte und Kurven sind die Mittelwerte aus jeweils 6 Proben.

Die folgenden Beispiele dienen der Erläuterung der Erfindung.

### Beispiel 1:

Tramadolpellets mit einem Wirkstoffgehalt von 55 Gew.% wurden durch wäßrige Granulation mit mikrokristalliner Cellulose und niedrig substituierter Hydroxypropylcellulose und anschließender Extrusion/Spheronisation hergestellt. Die Pellets mit einer Größe von 800-1250 µm werden getrocknet und anschließend in der Wirbelschicht bei einer Zulufttemperatur von 60°C zuerst mit 3 Gew.% Hydroxypropylmethylcellulose und Talkum als Subcoat und anschließend mit 11 Gew.% Surelease® E-7-7050 als Retardüberzug befilmt. Die Filmaufträge sind in Gewichtsprozent bezogen auf das Ausgangsgewicht der Pellets bzw. der Pellets mit Subcoat angegeben.
Die Diclofenacpellets mit einem Wirkstoffgehalt von 37 Gew.% wurden durch wäßrige Granulation mit mikrokristalliner Cellulose und Lactosemonohydrat und anschließender Extrusion/Spheronisation hergestellt. Die Pellets mit einer Größe von 800 - 1250 µm wurden getrocknet und anschließend in der Wirbelschicht bei einer Zulufttemperatur von 60°C zuerst mit 1 Gew.% Hydroxypropylmethylcellulose als Subcoat und anschließend mit 13 Gew.% Surelease® E-7-7050 als Retardüberzug befilmt. Die Filmaufträge sind in Gewichtsprozent bezogen auf das Ausgangsgewicht der Pellets bzw. der Pellets mit Subcoat angegeben. Im Anschluß daran werden die Diclofenac retard Pellets getrocknet und im Trockenschrank bei 60°C 2 Stunden lang getempert.

Auf einer geeigneten Steckkapselmaschine wurden dann Hartgelatinekapseln der Größe 0 mit 160 mg der beschriebenen Tramadol retard Pellets (= 75 mg Tramadol-HCl), 160 mg der beschriebenen Diclofenac retard Pellets (= 50 mg Diclofenac-Na) befüllt.
Zusammensetzung einer Kapsel Tramadol-Diclofenac retard 75/50 mg:

| **Zusammensetzung** | **pro Kapsel** |
|---|---|
| **Tramadol retard Pellets (Restfeuchte: 2,5%)** | **160 mg** |
| Tramadol-HCl | 75,0 mg |
| Mikrokristalline Cellulose (Avicel® PH 105 von FMC) | 31,4 mg |
| Niedrig substituierte Hydroxypropylcellulose (I-HPC® LH 31 von ShinEtsu) | 30,0 mg |
| Opadry® OY 29020 clear (Colorcon) | 2,9 mg |
| Talkum | 1,2 mg |
| Surelease® E-7-7050 (Colorcon) (Trockensubstanzanteil) | 15,5 mg |
| | |
| **Diclofenac retard Pellets (Restfeuchte: 3,6%)** | **160 mg** |
| Diclofenac-Na | 50,0 mg |
| Mikrokristalline Cellulose (Avicel® PH 105 von FMC) | 75,0 mg |
| Lactose. H₂O | 10,1 mg |
| Opadry® OY 29020 clear (Colorcon) | 1,4 mg |
| Surelease® E-7-7050 (Colorcon) (Trockensubstanzanteil) | 17,8 mg |

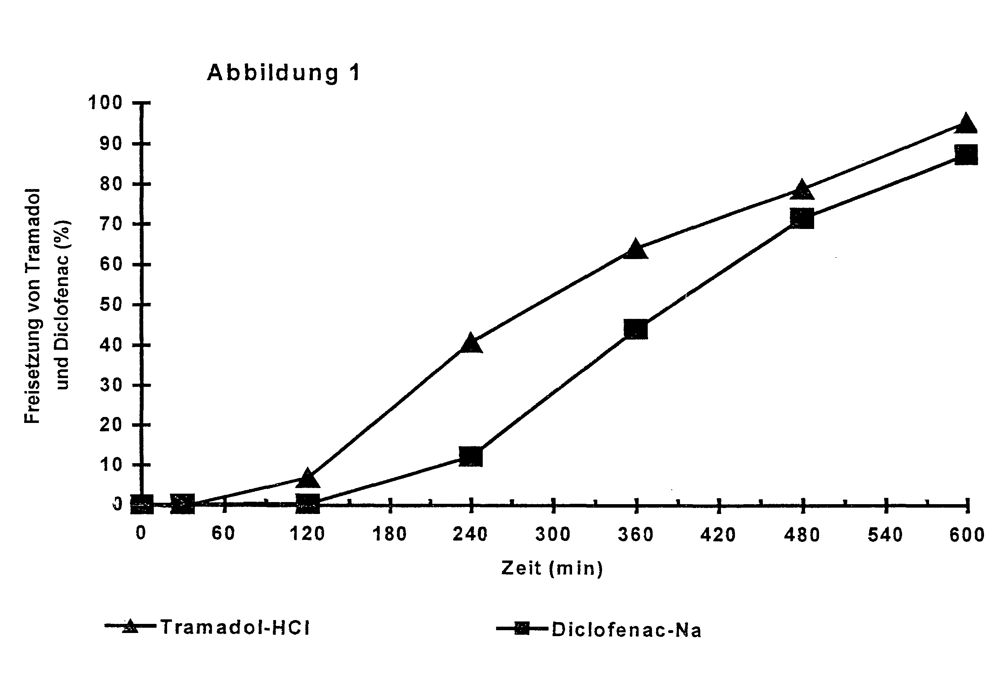

Das Freisetzungsprofil war wie folgt und ist in Abb. 1 dargestellt:

| Zeit in min | Freigesetzter Anteil in % | |
|---|---|---|
| | für Tramadol | für Diclofenac |
| 30 | 0,4 | 0,3 |
| 120 | 7 | 0,3 |
| 240 | 41 | 12 |
| 360 | 64 | 44 |
| 480 | 79 | 71 |
| 600 | 95 | 87 |

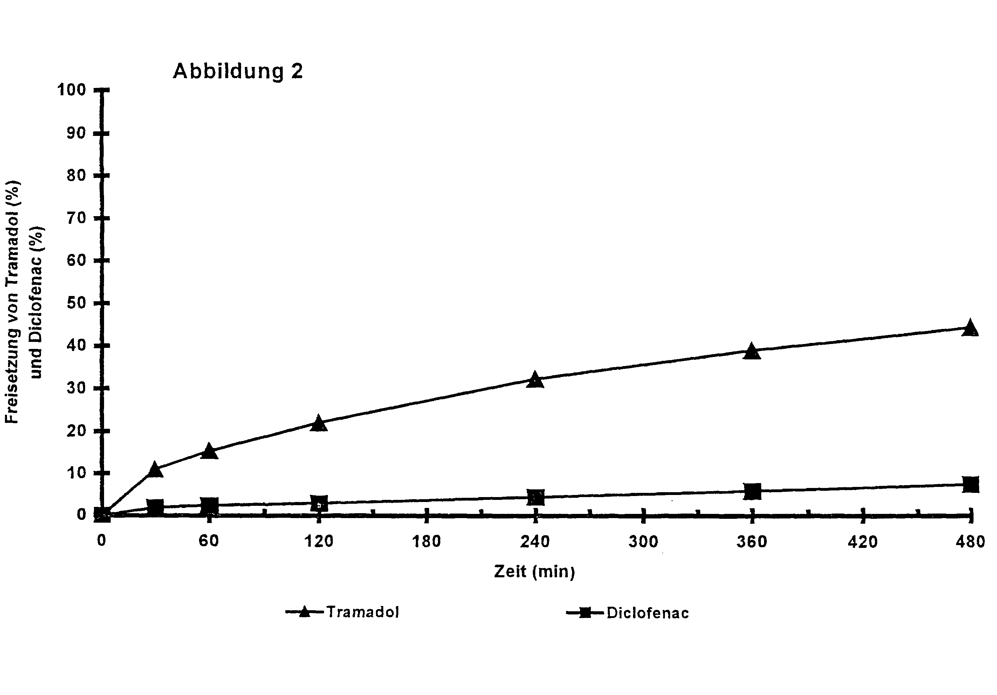

Die Abb. 2 zeigt das Freisetzungsprofil einer Matrixtablette mit einem Durchmesser von 12 mm, die 75 mg Tramadol-HCl und 50 mg Diclofenac-Na verpreßt in einer gemeinsamen hydrophilen Matrix aus Hydroxypropylmethylcellulose enthält. Ein Vergleich der Abb. 1 mit Abb. 2 zeigt, daß die freigesetzte Menge der Wirkstoffe Tramadol und Diclofenac aus der erfindungsgemäßen oralen Applikationseinheit nach 8 Stunden deutlich größer ist als die Freisetzung aus den sogenannten gemeinsamen Matrixtabletten.

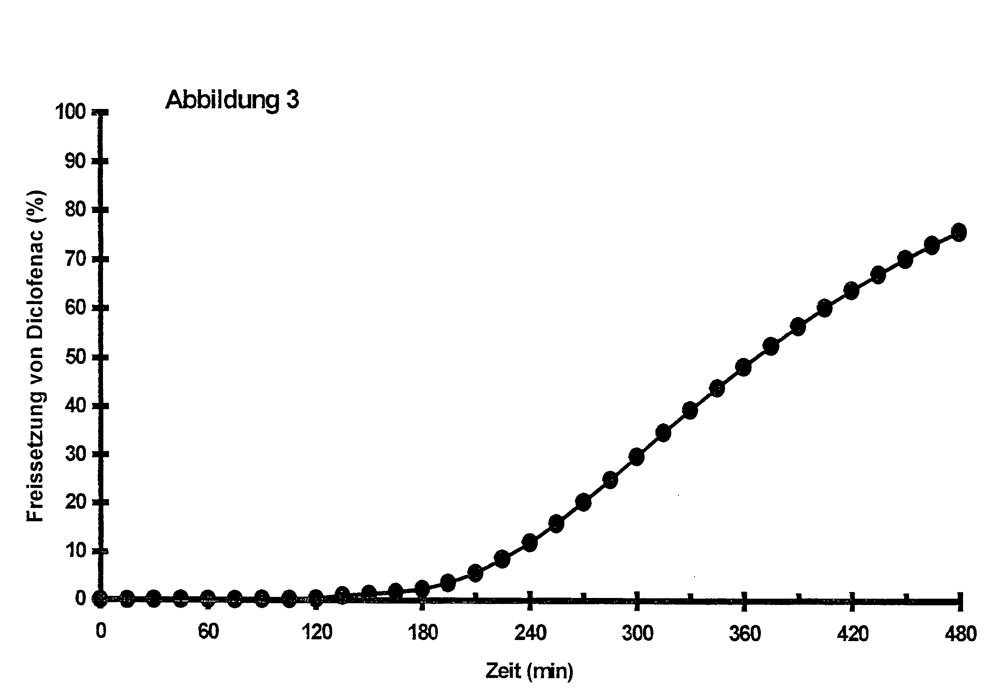

Abb. 3 zeigt die Diclofenac Freisetzung aus Diclofenac retard Pellets, die mit einem 1 % Gew.%-igen Subcoat aus Hydroxypropylmethylcellulose (Opadry® OY 29020, analog zu Beispiel 1) und einem 13 Gew.%-igen Surelease®-7-7050 Überzug befilmt sind. Abb. 4 zeigt die Freisetzung von Tramadol aus Tramadol retard Pellets mit einem 3 Gew.%-igen Subcoat aus Hydroxypropylmethylcellulose (Opadry® OY 29020, analog zu Beispiel 1) und Talkum und einem 11 Gew.%-igen Surelease® 7-7050 Überzug.

Ein Vergleich der Abb. 1 mit den Abb. 3 und 4 zeigt, daß die freigesetzten Mengen und die Freisetzungsprofile von Tramadol und Diclofenac aus den erfindungsgemäßen oralen Applikationseinheiten den Mengen und Freisetzungsprofilen aus den jeweils nur Tramadol- oder nur Diclofenac-haltigen Formen entsprechen.

### Beispiel 2:

Tramadol retard Pellets und Diclofenac retard Pellets wurden analog zu Beispiel 1 hergestellt. Tramadol Initialdosispellets wurden analog zu den retardierten Tramadolpellets hergestellt, aber nicht mit dem Surelease® E-7-7050 Überzug sondern lediglich mit 3% Subcoat aus Opadry® OY 29020 clear und Talkum befilmt. Die drei Pelletsorten wurden in einem Bohle Containermischer 10 Minuten lang miteinander gemischt.
368 mg Pellets, entsprechend einer Dosis von 100 mg Tramadolhydrochlorid und 50 mg Diclofenac-Na, wurden zuerst mit 30 mg Crospovidon und danach mit 330,6 mg Cellactose® und 7,4 mg Magnesiumstearat gemischt und zu 7 x 14 mm Tabletten mit Bruchkerbe und einem Gewicht von 736 mg verpreßt. Diese zerfallen in wässrigem Medium wieder in die einzelnen Pellets.

| **Zusammensetzung** | **pro Tablette** |
|---|---|
| **Tramadol retard Pellets (Restfeuchte: 2,5%)** | **160 mg** |
| Tramadol-HCl | 75,0 mg |
| Mikrokristalline Cellulose (Avicel® PH 105 von FMC) | 31,4 mg |
| Niedrig substituierte Hydroxypropylcellulose (I-HPC® LH 31 von ShinEtsu) | 30,0 mg |
| Opadry® OY 29020 clear (Colorcon) | 2,9 mg |
| Talkum | 1,2 mg |
| Surelease® E-7-7050 (Colorcon) (Trockensubstanzanteil) | 15,5 mg |
| | |
| **Tramadol Initialdosis Pellets (Restfeuchte: 2,5%)** | **48 mg** |
| Tramadol-HCl | 25,0 mg |
| Mikrokristalline Cellulose (Avicel PH® 105 von FMC) | 10,5 mg |
| Niedrig substituierte Hydroxypropylcellulose (I-HPC® LH 31 von ShinEtsu) | 10,0 mg |
| Opadry® OY 29020 clear (Colorcon) | 0,9 mg |
| Talkum | 0,4 mg |
| | |
| **Diclofenac retard Pellets (Restfeuchte: 3,6%)** | **160 mg** |
| Diclofenac-Na | 50,0 mg |
| Mikrokristalline Cellulose (Avicel® PH 105 von FMC) | 75,0 mg |
| Lactose. H₂O | 10,1 mg |
| Opadry® OY 29020 clear (Colorcon) | 1,4 mg |
| Surelease® E-7-7050 (Colorcon) (Trockensubstanzanteil) | 17,8 mg |
| | |
| Cellactose® (Meggle) | 330,6 mg |
| Crospovidon (Kollidon® CL von BASF) | 30 mg |
| Magnesiumstearat | 7,4 mg |
| **Gesamt** | **736 mg** |

Das Freisetzungsprofil war wie folgt:

| Zeit in min | Freigesetzter Anteil in % | |
|---|---|---|
| | für Tramadol | für Diclofenac |
| 30 | 28 | 0 |
| 120 | 35 | 0 |
| 240 | 62 | 20 |
| 360 | 78 | 40 |
| 480 | 89 | 78 |
| 600 | 100 | 98 |

### Beispiel 3:

Tramadolpellets mit einem Wirkstoffgehalt von 55 Gew.% wurden durch wäßrige Granulation mit mikrokristalliner Cellulose und niedrig substituierter Hydroxypropylcellulose und anschließender Extrusion/Spheronisation hergestellt. Die Pellets mit einer Größe von 800 - 1250 µm wurden getrocknet und anschließend in der Wirbelschicht bei einer Zulufttemperatur von 60°C mit 15 Gew.% Retardüberzug, bezogen auf das Ausgangsgewicht der Pellets, befilmt. Die getrockneten Tramadol retard Pellets wurden anschließend zur Einstellung des Freisetzungsprofiles weitere 2 h bei 60°C im Trockenschrank getrocknet, bevor sie mit einem Overcoat aus 0,6 Gew.% Hydroxypropylmethylcellulose, bezogen auf das Ausgangsgewicht der Pellets mit Retardüberzug, überzogen wurden. Die Diclofenacpellets mit einem Wirkstoffgehalt von 37 Gew.% werden durch wäßrige Granulation mit mikrokristalliner Cellulose und Lactosemonohydrat und anschließender Extrusion/Spheronisation hergestellt. Die getrockneten Pellets mit einer Größe von 800 - 1250 µm wurden dann in der Wirbelschicht bei 60°C Zulufttemperatur mit 16 Gew.% Retardüberzug, bezogen auf das Ausgangsgewicht der Pellets, befilmt. Die getrockneten Diclofenac retard Pellets wurden dann im Trockenschrank bei 60°C für 24 Stunden getempert.

Hartgelatinekapseln der Größe 0 werden mit 216 mg Tramadol retard Pellets (= 100 mg Tramadol-HCl und 162 mg Diclofenac retard Pellets (= 50 mg Diclofenac-Na) befüllt.

| **Zusammensetzung** | **Pro Kapsel** |
|---|---|
| **Tramadol retard Pellets (Restfeuchte: 2,5%)** | **216 mg** |
| Tramadol-HCl | 100,0 mg |
| Mikrokristalline Cellulose (Avicel® PH 105) | 42,0 mg |
| niedrig substituierte Hydroxypropylcellulose (I-HPC® LH 31) | 40,0 mg |
| Aquacoat® ECD 30 (Trockensubstanzanteil) | 18,6 mg |
| Dibutylsebacat | 4,4 mg |
| Talkum | 4,3 mg |
| Tween 80 | 0,002 mg |
| Opadry® OY 29020 clear | 1,3 mg |
| | |
| **Diclofenac retard Pellets (Restfeuchte: 3,3%)** | **162 mg** |
| Diclofenac-Na | 50,0 mg |
| Mikrokristalline Cellulose (Avicel® PH 105) | 75,0 mg |
| Lactose. H₂O | 10,1 mg |
| Aquacoat® ECD 30 (Trockensubstanzanteil) | 14,0 mg |
| Opadry® OY 29020 clear | 2,0 mg |
| Dibutylsebacat | 3,0 mg |
| Talkum | 2,6 mg |
| Tween 80 | 0,002 mg |

Das Freisetzungsprofil war wie folgt:

| Zeit in min | Freigesetzter Anteil in % | |
|---|---|---|
| | für Tramadol | für Diclofenac |
| 120 | 43 | 1 |
| 240 | 86 | 39 |
| 360 | 94 | 59 |
| 480 | 98 | 72 |

### Beispiel 4:

Tramadolhydrochlorid und mikrokristalline Cellulose wurden mit einer wässrigen Lösung von Povidon® K30 granuliert, getrocknet, gesiebt und nach Abmischen mit Magnesiumstearat zu Mikrotabletten mit einem Gewicht von 15,0 mg und einem Durchmesser von 3 mm Durchmesser verpreßt.

Die Mikrotabletten wurden bei 60°C Zulufttemperatur zuerst mit 2 Gew.% Subcoat aus Opadry® OY 29020 clear, bezogen auf das Gewicht der Tablettenkerne und anschließend mit 8 Gew.% Retardüberzug, bezogen auf das Gewicht der Tabletten mit Subcoat, überzogen. Das Endgewicht der Mikrotablette beträgt 16,6 mg.

Zusammensetzung einer Tramadol retard Mikrotablette

| | |
|---|---|
| Tramadolhydrochlorid | 10,0 mg |
| Mikrokristalline Cellulose (Avicel® PH 101 von FMC) | 4,0 mg |
| Povidon® K30 | 0,8 mg |
| Magnesiumstearat | 0,2 mg |
| Opadry® OY 29020 clear | 0,3 mg |
| Aquacoat® ECD 30 (Trockensubstanzanteil) | 1,0 mg |
| Dibutylsebacat | 0,3 mg |
| Gesamt | 16,6 mg |

Diclofenac Tabletten wurden analog zu den Tramadol Mikrotabletten hergestellt und ebenfalls zu Mikrotabletten mit einem Gewicht von 15 mg und einem Durchmesser von 3 mm verpreßt. Die Mikrotabletten werden mit 8% Polyacrylat-Dispersion magensaftresistent überzogen.

Zusammensetzung einer magensaftresistenten Diclofenac Mikrotablette

| | |
|---|---|
| Diclofenac-Na | 10,0 mg |
| Mikrokristalline Cellulose (Avicel® PH 101 von FMC) | 4,0 mg |
| Povidon® K30 | 0,8 mg |
| Magnesiumstearat | 0,2 mg |
| Eudragit® L 30 D (Trockensubstanzanteil) | 1,0 mg |
| Triethylcitrat | 0,1 mg |
| Talcum | 0,1 mg |
| Gesamt | 16,2 mg |

10 Tramadol retard Mikrotabletten und 5 Diclofenac Mikrotabletten mit magensaftresistentem Überzug werden in Hartgelatinekapseln der Größe 0 abgefüllt.

Das Freisetzungsprofil war wie folgt:

| Zeit in min | Freigesetzter Anteil in % | |
|---|---|---|
| | Für Tramadol | für Diclofenac |
| 120 | 11 | 0 |
| 240 | 37 | 82 |
| 360 | 64 | 96 |
| 480 | 98 | 99 |

## Patentansprüche

1. Orale Applikationseinheit enthaltend die Wirkstoffe Tramadol und Diclofenac und/oder deren jeweilige physiologisch verträgliche Salze, **dadurch gekennzeichnet, daß** die beiden Wirkstoffe in jeweils separat formulierten Untereinheiten in derselben Applikationseinheit vorliegen.

2. Orale Applikationseinheit nach Anspruch 1, **dadurch gekennzeichnet, daß** sie als physiologisch verträgliches Salz des Tramadols Tramadolhydrochlorid, Tramadolhydrobromid, Tramadolsulfat, Tramadolphosphat, Tramadolfumarat, Tramadolsuccinat, Tramadolmaleat, Tramadolnitrat, Tramadolacetat, Tramadolpropionat, Tramadolmalonat, Tramadolcitrat, Tramadoltartrat, Tramadolbenzoat, Tramadolsalicylat, Tramadolphthalat und/oder Tramadolnicotinat, bevorzugt Tramadolhydrochlorid und als physiologisch verträgliches Salz des Diclofenacs Diclofenac-Natrium, Diclofenac-Kalium, Diclofenac-Kalzium, Diclofenac-Magnesium und/oder Diclofenac-Colestyramin, bevorzugt Diclofenac-Natrium enthält.

3. Orale Applikationseinheit nach Anspruch 1 oder 2, **dadurch gekennzeichnet, daß** sie die Wirkstoffe Tramadol und Diclofenac in einem Mengenverhältnis von ≤1:4 bis 4:≤1, bevorzugt im Mengenverhältnis von 0,5:1 bis 3:1 und besonders bevorzugt im Mengenverhältnis von 1:1 bis 2,5:1 enthält.

4. Orale Applikationseinheit nach einem oder mehreren der Ansprüche 1 bis 3, **dadurch gekennzeichnet, daß** die Untereinheiten in derselben oder unterschiedlichen multipartikulärer Form vorliegen.

5. Orale Applikationseinheit nach Anspruch 4, **dadurch gekennzeichnet, daß** die Untereinheiten in Form von Mikrotabletten, Mikrokapseln, Ionenaustauscherresinaten, Granulaten, Wirkstoffkristallen oder Pellets vorliegen.

6. Orale Applikationseinheit nach Anspruch 5, **dadurch gekennzeichnet, daß** die Untereinheiten in Form von durch Extrusion und/oder Spheronisation hergestellten Pellets oder Aufbaupellets vorliegen.

7. Orale Applikationseinheit nach einem oder mehreren der Ansprüche 1 bis 6, **dadurch gekennzeichnet, daß** zumindest einer der beiden Wirkstoffe in einer retardierten, multipartikulären Form, bevorzugt beide Wirkstoffe in einer retardierten, multipartikulären Form vorliegen.

8. Orale Applikationseinheit nach Anspruch 7, **dadurch gekennzeichnet, daß** die Retardierung durch einen retardierenden Überzug, Fixierung an einem Ionenaustauscherharz, Einbettung in eine retardierende Matrix oder einer Kombination daraus erfolgt ist.

9. Orale Applikationseinheit nach Anspruch 8, **dadurch gekennzeichnet, daß** der Überzug auf einem wasserunlöslichen Polymeren oder Wachs basiert.

10. Orale Applikationseinheit nach Anspruch 9, **dadurch gekennzeichnet, daß** als wasserunlösliches Polymeres ein Polyacrylharz oder Cellulosederivat, vorzugsweise Alkylcellulose, eingesetzt ist.

11. Orale Applikationseinheit nach Anspruch 10, **dadurch gekennzeichnet, daß** Ethylcellulose und/oder ein Poly(meth)acrylat als Polymeres eingesetzt ist.

12. Orale Applikationseinheit nach Anspruch 7, **dadurch gekennzeichnet, daß** die Retardierung der multipartikulären Formen durch Einbettung in eine retardierende Matrix erreicht ist.

13. Orale Applikationseinheit nach einem oder mehreren der Ansprüche 7 bis 12, **dadurch gekennzeichnet, daß** neben der retardierten Form der Wirkstoffe mindestens einer der Wirkstoffe auch in einer unretardierten Form vorliegt.

14. Orale Applikationseinheit nach einem oder mehreren der Ansprüche 1 bis 13. **dadurch gekennzeichnet, daß** sie als ein Sachet, eine Kapsel oder eine Tablette vorliegt.

15. Orale Applikationseinheit nach einem oder mehreren der Ansprüche 1 bis 13, **dadurch gekennzeichnet, daß** sie als Kapsel oder als Pellettablette vorliegt.

16. Orale Applikationseinheit nach Anspruch 14 oder 15, **dadurch gekennzeichnet, daß** die Tablette eine schnell zerfallende Tablette ist.

17. Orale Applikationseinheit nach einem oder mehreren der Ansprüche 14 bis 16, **dadurch gekennzeichnet, daß** sie mindestens eine Sprengschicht aufweist, die bei Kontakt mit wäßrigen Körperflüssigkeiten die Ablösung der Untereinheiten voneinander bewirkt.

18. Orale Applikationseinheit nach einem der Ansprüche 14 bis 17, **dadurch gekennzeichnet, daß** die Tablette eine Bruchkerbe aufweist.

19. Orale Applikationseinheit nach einem der Ansprüche 14 bis 18, **dadurch gekennzeichnet, daß** die Tablette einen magensaftresistenten Überzug aufweist.

20. Orale Applikationseinheit für eine zweimalige Applikation pro Tag nach einem oder mehreren der Ansprüche 1 bis 19, **dadurch gekennzeichnet, daß** das Tramadol bzw. das Diclofenac innerhalb von 8 Stunden ≥ 70 Gew.% bzw. ≥ 60 Gew. % freigesetzt wird.

21. Orale Applikationseinheit für eine einmalige Applikation pro Tag nach einem oder mehreren der Ansprüche 1 bis 20, **dadurch gekennzeichnet, daß** das Tramadol bzw. das Diclofenac innerhalb von 16 Stunden ≥ 70 Gew. % bzw. ≥ 60 Gew.% freigesetzt wird.

## Claims

1. Oral application unit containing the active substances Tramadol and Diclofenac and/or their respective physiologically compatible salts, **characterised in that** the two active substances are present in subunits separately formulated in each case, in the same application unit.

2. Oral application unit according to claim 1, **characterised in that** it contains as physiologically compatible salt of Tramadol: Tramadol hydrochloride, Tramadol hydrobromide, Tramadol sulfate, Tramadol phosphate, Tramadol fumarate, Tramadol succinate, Tramadol maleate, Tramadol nitrate, Tramadol acetate, Tramadol propionate, Tramadol malonate, Tramadol citrate, Tramadol tartrate, Tramadol benzoate, Tramadol salicylate, Tramadol phthalate and/or Tramadol nicotinate, preferably Tramadol hydrochloride, and as physiologically compatible salt of Diclofenac: Diclofenac-sodium, Diclofenac-potassium, Diclofenac-calcium, Diclofenac-magnesium and/or Diclofenac-cholestyramine, preferably Diclofenac-sodium.

3. Oral application unit according to claim 1 or 2, **characterised in that** the active substances Tramadol and Diclofenac are contained in a quantitative ratio of ≤1:4 to 4:≤1, preferably in a quantitative ratio of 0.5:1 to 3:1, and particularly preferably in a quantitative ratio of 1:1 to 2.5:1.

4. Oral application unit according to one or more of claims 1 to 3, **characterised in that** the subunits are present in the same or different multiparticulate form.

5. Oral application unit according to claim 4, **characterised in that** the subunits are present in the form of microtablets, microcapsules, ion-exchange resinates, granules, active substance crystals or pellets.

6. Oral application unit according to claim 5, **characterised in that** the subunits are present in the form of pellets or composite pellets produced by extrusion and/or spheronisation.

7. Oral application unit according to one or more of claims 1 to 6, **characterised in that** at least one of the two active substances is present in a retarded (delayed release), multiparticulate form, and preferably both active substances are present in a retarded, multiparticulate form.

8. Oral application unit according to claim 7, **characterised in that** the retarded effect is produced by a retarded coating, binding to an ion-exchange resin, embedding in a retarded matrix or a combination thereof.

9. Oral application unit according to claim 8, **characterised in that** the coating is based on a water-insoluble polymer or wax.

10. Oral application unit according to claim 9, **characterised in that** a polyacrylic resin or cellulose derivative, preferably alkylcellulose, is used as water-insoluble polymer.

11. Oral application unit according to claim 10, **characterised in that** ethylcellulose and/or a poly(meth)acrylate is used as polymer.

12. Oral application unit according to claim 7, **characterised in that** the retarded effect of the multiparticulate forms is achieved by embedding in a retarded matrix.

13. Oral application unit according to one or more of claims 7 to 12, **characterised in that** in addition to the.retarded form of the active substances, at least one of the said active substances is also present in a non-retarded form.

14. Oral application unit according to one or more of claims 1 to 13, **characterised in that** it is present as a sachet, a capsule or a tablet.

15. Oral application unit according to one or more of claims 1 to 13, **characterised in that** it is present as a capsule or as a pellet tablet.

16. Oral application unit according to claim 14 or 15, **characterised in that** the tablet is a rapidly decomposing tablet.

17. Oral application unit according to one or more of claims 14 to 16, **characterised in that** it has at least one release layer that effects the dissociation of the subunits from one another on contact with aqueous body fluids.

18. Oral application unit according to one of claims 14 to 17, **characterised in that** the tablet has a score mark.

19. Oral application unit according to one of claims 14 to 18, **characterised in that** the tablet has a gastric juice-resistant coating.

20. Oral application unit for a twice daily application according to one or more of claims 1 to 19, **characterised in that** the Tramadol and the Diclofenac is released in an amount of ≥70 wt.% and ≥60 wt.% respectively within 8 hours.

21. Oral application unit for a single daily application according to one or more of claims 1 to 20, **characterised in that** the Tramadol and the Diclofenac is released in an amount of ≥70 wt.% and ≥60 wt.% respectively within 16 hours.

## Revendications

1. Unité d'administration orale contenant les substances actives tramadol et diclofénac et/ou leurs sels acceptables du point de vue physiologique, **caractérisée en ce que** les deux substances actives sont présentes chacune en sous-unités formulées séparément dans la même unité d'administration.

2. Unité d'administration orale suivant la revendication 1, **caractérisée en ce qu'**elle contient comme sel du tramadol acceptable du point de vue physiologique, du chlorhydrate de tramadol, du bromhydrate de tramadol, du sulfate de tramadol, du phosphate de tramadol, du fumarate de tramadol, du succinate de tramadol, du maléate de tramadol, du nitrate de tramadol, de l'acétate de tramadol, du propionate de tramadol, du malonate de tramadol, du citrate de tramadol, du tartrate de tramadol, du benzoate de tramadol, du salicylate de tramadol, du phtalate de tramadol et/ou du nicotinate de tramadol, de préférence du chlorhydrate de tramadol, et comme sel de diclofénac acceptable du point de vue physiologique, du diclofénac-sodium, du diclofénac-potassium, du diclofénac-calcium, du diclofénac-magnésium et/ou du diclofénac-colestyramine, de préférence du diclofénac-sodium.

3. Unité d'administration orale suivant la revendication 1 ou 2, **caractérisée en ce qu'**elle contient les substances actives tramadol et diclofénac dans un rapport quantitatif de ≤ 1:4 à 4:≤ 1, avantageusement dans le rapport quantitatif de 0,5:1 à 3:1 et notamment dans le rapport quantitatif de 1:1 à 2,5 :1.

4. Unité d'administration orale suivant une ou plusieurs des revendications 1 à 3, **caractérisée en ce que** les sous-unités sont présentes sous la même forme multiparticulaire ou sous des formes multiparticulaires différentes.

5. Unité d'administration orale suivant la revendication 4, **caractérisée en ce que** les sous-unités sont présentes sous forme de microcomprimés, de microcapsules, de combinaisons avec des résines échangeuses d'ions, de granulés, de cristaux de substances actives ou de pellets.

6. Unité d'administration orale suivant la revendication 5, **caractérisée en ce que** les sous-unités sont présentes sous forme de pellets ou de pellets structurés produits par extrusion et/ou par sphéronisation.

7. Unité d'administration orale suivant une ou plusieurs des revendications 1 à 6, **caractérisée en ce qu'**au moins l'une des deux substances actives et de préférence les deux substances actives sont présentes sous une forme multiparticulaire retardée.

8. Unité d'administration orale suivant la revendication 7, **caractérisée en ce que** le retardement est réalisé par un revêtement retardateur, la fixation à une résine échangeuse d'ions, l'enrobage dans une matrice retardatrice, ou une combinaison de ces possibilités.

9. Unité d'administration orale suivant la revendication 8, **caractérisée en ce que** le revêtement est à base d'un polymère ou d'une cire insolubles dans l'eau.

10. Unité d'administration orale suivant la revendication 9, **caractérisée en ce qu'**on utilise comme polymère insoluble dans l'eau une résine polyacrylique ou un dérivé de cellulose, de préférence une alkylcellulose.

11. Unité d'administration orale suivant la revendication 10, **caractérisée en ce qu'**on utilise comme polymère l'éthylcellulose et/ou un polymère d'acrylate ou de méthacrylate.

12. Unité d'administration orale suivant la revendication 7, **caractérisée en ce que** le retardement des formes multiparticulaires est effectué par enrobage dans une matrice retardatrice.

13. Unité d'administration orale suivant une ou plusieurs des revendications 7 à 12, **caractérisée en ce que** l'une au moins des substances actives est aussi présente sous une forme non retardée, à côté de la forme retardée des substances actives.

14. Unité d'administration orale suivant une ou plusieurs des revendications 1 à 13, **caractérisée en ce qu'**elle est présente sous forme d'un sachet, d'une gélule ou d'un comprimé.

15. Unité d'administration orale suivant une ou plusieurs des revendications 1 à 13, **caractérisée en ce qu'**elle est présente sous forme d'une gélule ou d'un comprimé de pellets.

16. Unité d'administration orale suivant la revendication 14 ou 15, **caractérisée en ce que** le comprimé est un comprimé à désintégration rapide.

17. Unité d'administration orale suivant une ou plusieurs des revendications 14 à 16, **caractérisée en ce qu'**elle présente au moins une couche de désintégration qui produit au contact de liquides corporels aqueux la séparation des sous-unités les unes des autres.

18. Unité d'administration orale suivant l'une des revendications 14 à 17, **caractérisée en ce que** le comprimé présente une encoche de rupture.

19. Unité d'administration orale suivant l'une des revendications 14 à 18, **caractérisée en ce que** le comprimé présente un revêtement résistant au suc gastrique.

20. Unité d'administration orale pour une administration deux fois par jour selon une ou plusieurs des revendications 1 à 19, **caractérisée en ce que** le tramadol ou respectivement le diclofénac est libéré en 8 h en proportion supérieure ou égale à 70 % en poids ou respectivement supérieure ou égale à 60 % en poids.

21. Unité d'administration orale pour une administration une fois par jour selon une ou plusieurs des revendications 1 à 20, **caractérisée en ce que** le tramadol ou respectivement le diclofénac est libéré en 16 h en proportion supérieure ou égale à 70 % en poids ou respectivement supérieure ou égale à 60 % en poids.
